# EUROPEAN PATENT APPLICATION

(11) **EP 2 153 858 A1**
(43) Date of publication of application: **17.02.2010**
(21) Application number: 08162000.7
(22) Date of filing: 07.08.2008
(51) Int. Cl.: A61M 16/12, A63H 27/10, B64B 1/40, C01B 23/00, F17C 1/00

(54) **Improved helium balloon lifting-gas**

(71) Applicant: Air Products and Chemicals, Inc., Allentown, PA 18195-1501 (US)
(72) Inventor: TASSELL, Andrew Duncan, Overton, Hampshire RG25 3PE (GB); DOWNIE, Neil Alexander, Odiham, Hampshire RG29 1JN (GB)
(74) Representative: Burford, Anthony Frederick

(57) **Abstract**

The risk of asphyxiation with helium used as a lifting gas for balloons is reduced by providing the helium as a breathable mixture with oxygen and 2 to 5 volume % carbon dioxide.

## Description

The present invention relates to helium-containing lifting gases for filling balloons and has particular, but not exclusive, application to the filling of party balloons. It provides compressed cylinders of the lifting gas and balloons filled with the lifting gas and, in one embodiment, a lifting gas consisting essentially of helium, oxygen and carbon dioxide.

Party balloons are flexible bags, usually formed of foil, latex or plastics, that are inflated with air or other gas to provide a child's toy or decoration or for advertising purposes. When filled with a gas sufficiently lighter than air (lifting gas), they are air-buoyant and float, usually ascending in still air. Helium is the gas of choice as a lifting gas and is widely used for that purpose. It is the second lightest element, colourless, odourless and inert. It is not toxic and is used in admixture with oxygen (Heliox), with air (Heliair), with oxygen and nitrogen (Trimix), or with oxygen and hydrogen (Hydreliox) in underwater diving systems. It also is used with air or oxygen for various medical applications such as treating severe respiratory obstruction, to prevent atelectasis (i.e. partial or complete lung collapse) and in measuring lung function. However, it is an asphyxiant in high concentrations and, in recent years, inhalation of helium has been informally suggested as an effective and peaceful means of self-suffocation for terminally ill patients.

It is well known that inhalation of helium changes the timbre of the voice resulting in a high-pitched voice similar to that of the cartoon characters Donald Duck and the Chipmunks. It is a relatively widespread practice to inhale helium from balloons or even directly from helium gas cylinders to produce this effect. However, the attendant risk of asphyxiation is either unknown or ignored. The risk is particularly high when breathing helium from cylinders in which helium is supplied for use as a lifting gas because the cylinders contain gas of up to about 300 bar (30 MPa) pressure and the human lung can be irreversibly damaged by pressures as low as about 1 bar (0.1 MPa). Further, because the cylinders usually contain substantially more helium than required for individual balloons and issues as a high pressure stream, the user is likely to inhale substantially more helium than from a balloon. However, there have been reports of suffocation by persons breathing helium from large party balloons used for advertising purposes.

The object of the present invention is to reduce the risk of asphyxiation using helium as a lifting gas to provide air-buoyancy to party or other balloons.

The term "balloon" except when qualified by "party" is used in this application to include all types of air-buoyant balloons including party balloons; research balloons; weather balloons; aerostats such as rigid and non-rigid airships, helikites and observation balloons; and barrage balloons. The term "air-buoyant" used herein means that, unless tethered, the balloon will ascend from ground level. The term "lifting gas" used herein means a gas sufficiently lighter than air to cause at least a balloon of negligible weight to ascend in still air.

It is known to reduce the cost of filling a balloon with helium by diluting the helium with air. This can be done by provision of cylinders of compressed helium mixed with up to about 10% air or by providing a venturi arrangement that uses the Bernoulli effect of a convergent-divergent pipe to create a suction to suck in air from the atmosphere to dilute the helium by as much as about 50%. However, it is not known to add to the gas mixture carbon dioxide in an amount in excess of that present in the air.

US-A-4807706 published in February 1989 and corresponding to EP-A-0301464 (published the same month) discloses the introduction into a confined inhabitable space of carbon dioxide and an inert gas, which can be helium, to lower the oxygen concentration to the range of about 8 to about 15 percent by volume while increasing the carbon dioxide content to the range of about 2 to about 5 percent by volume. The combination of reducing oxygen concentration and increasing carbon dioxide concentration in the gaseous environment of the confined space maintains human consciousness and mental acuity while extinguishing flame.

The present inventors have found that the risk of asphyxiation by a helium-containing lifting gas can be significantly reduced whilst retaining adequate air-buoyancy by using a breathable gas mixture containing helium, oxygen and about 2 to about 5 volume % carbon dioxide. The oxygen must be present in a sufficient amount that the gas is breathable, by which is meant that breathing the gas does not produce asphyxiation. Usually, this will require the presence of at least about 8% oxygen and, in practice, the amount of oxygen employed will be a balance between ease of breathing the gas and buoyancy requirements. The presence of the carbon dioxide not only increases the brain blood flow and brain oxygenation but also facilitates remote detection of leaks via detection of carbon dioxide infrared adsorption bands and reduces the rate at which gas is lost from latex balloons compared with pure helium.

In a first aspect, the present invention provides a compressed gas cylinder containing a breathable lifting gas mixture of helium, oxygen and about 2 to about 5 volume % carbon dioxide. The mixture can contain additional components provided they do not make the gas mixture asphyxiant or prevent the gas from use as a lifting gas. For example, small amounts of, for example, up to about 5%, and preferably no more than about 3%, hydrogen or methane could be present. Further, at least part of the oxygen can be provided as air, in which case the gas can contain a substantial proportion of nitrogen. However, the gas usually will consist essentially of helium, oxygen and carbon dioxide (i.e. contain only impurity or trace amounts of other gases) and a second aspect of the invention provides a breathable lifting gas consisting essentially of helium, oxygen and 2 to 5 volume % carbon dioxide.

The composition of the lifting gas is chosen to provide required lift for the balloon while the gas remains breathable. As is known, latex balloons are significantly lighter for the same volume as foil balloons, which are flatter in shape and have a heavier skin than latex balloons, and accordingly can accommodate higher levels of non-helium components. For example, 11 inch (30 cm) and larger latex balloons will fly acceptably with a helium content as low as about 40%, whereas standard 18 inch (45 cm) foil balloons usually require at least about 90% helium.

Preferably the helium content will be in excess of 70 volume % and usually the gas mixture will comprise at least about 75 volume % helium, about 8 to about 21 volume % oxygen and about 2 to about 5 volume % carbon dioxide. Preferably, the oxygen content is about 8 to about 15 volume %, especially about 10 to about 12 volume % and, independently, the preferred carbon dioxide content is about 2 to about 3 volume %. For example, the gas mixture can consists of about 87 volume % helium, about 10 volume % oxygen and about 3 volume % carbon dioxide.

Usually, the gas mixture will have a density of less than about 0.9 kg/m³ at normal temperature and pressure (NTP), which is 20°C (68°F) and 0.1 MPa (14.7 psia). Preferably, the density is less than about 0.6 kg/m³ and more preferably less than about 0.4 kg/m³ at NTP.

In a third aspect, the invention provides an air-buoyant balloon containing a breathable lifting gas mixture of the first or second aspect. As mentioned previously, the invention has particular application to party balloons but also has application to other air buoyant balloons.

The gas mixture can be prepared by conventional industrial gas techniques such as pressure-based or gravimetric methods of filling a compressed gas cylinder with correct amounts of each component gas. If necessary, the cylinders can be rolled after filling to ensure mixing.

### EXAMPLE

A gas cylinder was filled with a mixture of 87 volume % helium, 10 volume % oxygen and 3 volume % carbon dioxide at a pressure of 300 bar (30 MPa). Gas from the cylinder was used to fill 18" (45 cm) Mylar balloons. The balloons ascended at a satisfactory rate on release. When the gas mixture was breathed, it produced a slightly unpleasant taste but no difficulty in breathing.

It will be appreciated that the invention is not restricted to the details described above with reference to the preferred embodiments but that numerous modifications and variations can be made without departing from the spirit and scope of the invention as defined in the following claims.

## Claims

1. A compressed gas cylinder containing a breathable lifting gas mixture of helium, oxygen and 2 to 5 volume % carbon dioxide.

2. A compressed gas cylinder according to Claim 1, wherein the gas mixture consists essentially of helium, oxygen and carbon dioxide.

3. A breathable lifting gas consisting essentially of helium, oxygen and 2 to 5 volume % carbon dioxide.

4. A gas or compressed gas cylinder according to any one of Claims 1 to 3, wherein the helium content of the gas mixture is in excess of 70 volume %.

5. A gas or compressed gas cylinder according to any one of the preceding claims, wherein the gas mixture comprises at least 75 volume % helium, 8 to 21 volume % oxygen and 2 to 5 volume % carbon dioxide.

6. A gas or compressed gas cylinder according to any one of the preceding claims, wherein the gas mixture comprises 8 to 15 volume % oxygen.

7. A gas or compressed gas cylinder according to Claim 5, wherein the gas mixture comprises 10 to 12 volume % oxygen.

8. A gas or compressed gas cylinder according to any one of the preceding claims, wherein the gas mixture comprises 2 to 3 volume % carbon dioxide.

9. A gas or compressed gas cylinder according to Claim 2 or Claim 3, wherein the gas mixture consists of about 87 volume % helium, about 10 volume % oxygen and about 3 volume % carbon dioxide.

10. A gas or compressed gas cylinder according to any one of the preceding claims, wherein the gas mixture has a density of less than 0.9 kg/m³ at NTP (i.e. 20°C/68°F; 14.7 psia/0.1 MPa)

11. A gas or compressed gas cylinder according to Claim 10, wherein the gas mixture has a density of less than 0.6 kg/m³ at NTP

12. A gas or compressed gas cylinder according to Claim 11, wherein the gas mixture has a density of less than 0.4 kg/m³ at NTP.

13. An air-buoyant balloon containing a breathable lifting gas mixture of helium, oxygen and 2 to 5 volume % carbon dioxide.

14. A balloon according to Claim 13, wherein the gas mixture is as defined in any one of Claims 2 to 12.

15. A balloon according to Claim 13 or Claim 14 which is a party balloon.
